# EUROPEAN PATENT APPLICATION

(11) **EP 4 806 888 A1**
(43) Date of publication of application: **16.09.2026**
(21) Application number: 24915742.1
(22) Date of filing: 05.01.2024
(51) Int. Cl.: C07D 249/06, A61K 31/4192, A61P 25/00

(54) **METHOD FOR PREPARING NOVEL COMPOUND**

(71) Applicant: PRG S&Tech Inc., Busan 46274 (KR)
(72) Inventor: KIM, Bae-Hoon, Busan 46277 (KR); PARK, Bum-Joon, Busan 46241 (KR)
(74) Representative: Roos, Peter
(86) International application number: PCT/KR2024/000251
(87) International publication number: WO 2025/146850

(57) **Abstract**

The present invention relates to a method for preparing 4-(4-(2-(diethylamino)ethoxy)phenyl)-1-(4-methoxybenzyl)-1H-1,2,3-triazole-5-amine, which is a novel candidate material for NF2 treatment. Through an optimal preparation method, it is possible to prepare a compound with low impurities and high purity even at low manufacturing costs and within a short manufacturing time, and the compound prepared thereby can be used as a novel therapeutic agent for type 2 neurofibromatosis (NF2).

## Description

### [Technical Field]

The present disclosure relates to providing a method for preparing a novel compound, and more specifically, provides a method for preparing 4-(4-(2-(diethylamino)ethoxy)phenyl)-1-(4-methoxybenzyl)-1H-1,2,3-triazole-5-amine.

### [Background Art of the Invention]

Neurofibromatosis (NF) is a genetic disease affecting bone, soft tissue, skin, and the nervous system, and is classified into neurofibromatosis type 1 (NF1) and neurofibromatosis type 2 (NF2). Neurofibromatosis type 2 is a disease in which benign tumors arising from the eighth cranial nerve, resulting in symptoms such as hearing loss, tinnitus, and balance disorder, and is also referred to as vestibular schwannomas because it originate from Schwann cells within the central nervous system. The average age of onset of neurofibromatosis type 2 is 18 to 24 years, and it is known that bilateral vestibular schwannomas occur in almost all patients by the age of 30. In addition, schwannomas of cranial nerves and peripheral nerves, meningiomas, ependymomas, and, very rarely, astrocytomas may progress.

Neurofibromatosis type 2 is caused by mutations in the NF2 gene located on the long arm of chromosome 22 (22q12.2). The NF2 gene encodes a protein called merlin, and merlin is produced in Schwann cells surrounding nerve cells of the brain and spinal cord in the nervous system.

Meanwhile, Korean Patent Application Publication No. 10-2022-0128710 has discovered a novel compound for treating neurofibromatosis type 2 (NF2), and research on an optimal preparation method for the compound is continuing.

### [Description]

### [Challenge to solve]

An object of the present disclosure is to provide an optimal method for preparing 4-(4-(2-(diethylamino)ethoxy)phenyl)-1-(4-methoxybenzyl)-1H-1,2,3-triazole-5-amine.

### [Solution to the Problem]

In order to achieve the above purpose, the present disclosure provides a method for preparing 4-(4-(2-(diethylamino)ethoxy)phenyl)-1-(4-methoxybenzyl)-1H-1,2,3-triazole-5-amine, the method comprising: a) dissolving 4-MBC (4-methoxybenzyl chloride) in DMF (dimethylformamide), and then adding NaN₃ (Sodium azide) thereto and reacting the same for 1 to 3 hours to synthesize an intermediate; b-1) dissolving 4-HPA (4-hydroxyphenylacetonitrile) in THF (tetrahydrofuran), and then adding DEAECl.HCl (2-Diethylaminoethyl chloride hydrochloride) and water thereto and cooling the same to 5 to 20 °C; b-2) adding an aqueous NaOH solution to the reaction product of step b-1) and reacting the same for 2 to 4 hours to synthesize an intermediate; c) dissolving the intermediate of step a) and the intermediate of step b-2) in DMSO, and then adding KTB (Potassium tert-Butoxide; t-BuOK) thereto and reacting the same at 20 to 40 °C for 1 to 3 hours; and d) dissolving the reaction product of step c) in CH₂Cl₂ (dichloromethane), adding activated carbon thereto, stirring the same at 15 to 40 °C for 30 to 100 minutes, and filtering and washing the same.

### [Effects of the Invention]

The present disclosure relates to a method for preparing a novel compound for treating NF2, and may prepare a compound having high purity and yield while generating a small amount of impurities even with low preparation cost and preparation time through an optimal preparation method.

### [Brief Description of Drawings]

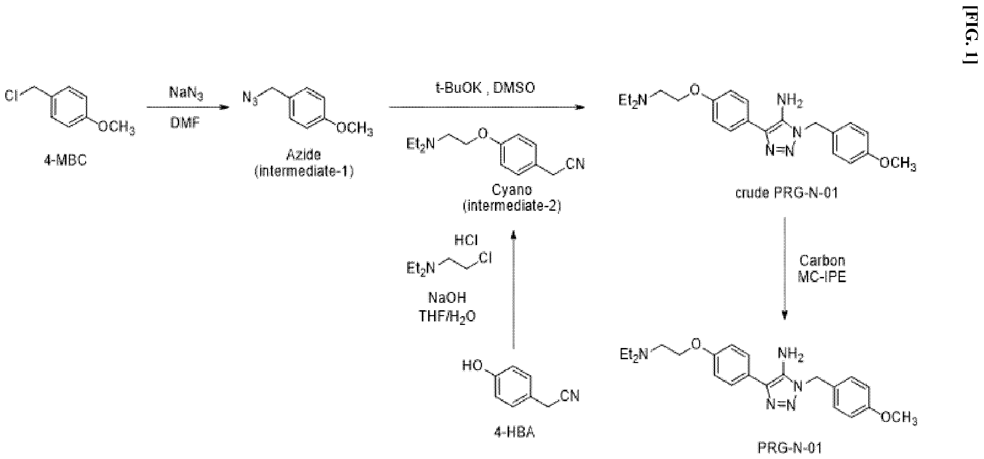

FIG. 1 shows a synthetic scheme of 4-(4-(2-(diethylamino)ethoxy)phenyl)-1-(4-methoxybenzyl)-1H-1,2,3-triazole-5-amine (hereinafter referred to as PRG-N-01).

### [Best Mode for Carrying Out the Invention]

Hereinafter, the present disclosure will be described in more detail.

The present disclosure provides a method for preparing 4-(4-(2-(diethylamino)ethoxy)phenyl)-1-(4-methoxybenzyl)-1H-1,2,3-triazole-5-amine, the method comprising: a) dissolving 4-MBC (4-methoxybenzyl chloride) in DMF (dimethylformamide), and then adding NaN₃ (Sodium azide) thereto and reacting the same for 1 to 3 hours to synthesize an intermediate; b-1) dissolving 4-HPA (4-hydroxyphenylacetonitrile) in THF (tetrahydrofuran), and then adding DEAECl.HCl (2-Diethylaminoethyl chloride hydrochloride) and water thereto and cooling the same to 5 to 20 °C; b-2) adding an aqueous NaOH solution to the reaction product of step b-1) and reacting the same for 2 to 4 hours to synthesize an intermediate; c) dissolving the intermediate of step a) and the intermediate of step b-2) in DMSO, and then adding KTB (Potassium tert-Butoxide; t-BuOK) thereto and reacting the same at 20 to 40 °C for 1 to 3 hours; and d) dissolving the reaction product of step c) in CH₂Cl₂ (dichloromethane), adding activated carbon thereto, stirring the same at 15 to 40 °C for 30 to 100 minutes, and filtering and washing the same.

The reaction of step a) may be performed at 15 to 60 °C, and preferably may be 25 to 30 °C.

The amount of NaN₃ used in step a) may be 1.0 to 3.0 eq, preferably 1.1 to 2.2 eq, and more preferably 1.5 eq.

The amount of DEAECl.HCl used in step b-1) may be 1.0 to 1.5 eq, and preferably may be 1.2 eq.

The reaction of step b-2) may be performed at 40 to 70 °C, and preferably may be 60 to 65 °C.

The amount of NaOH used in step b-2) may be 2.5 to 4 eq, and preferably may be 3.0 eq.

The aqueous NaOH solution of step b-2) may be a 20 to 50 %(w/v) aqueous NaOH solution, preferably 22 to 45 %(w/v), and more preferably 22.5 %(w/v).

The amount of KTB used in step c) may be 1.0 to 3.0 eq, and preferably may be 2.0 eq.

In step d), a volume ratio of the reaction product of step c) to CH₂Cl₂ (dichloromethane) may be 1 : 1.5 to 4.0, and preferably may be 1 : 3.

### [Mode for Carrying Out the Invention]

Hereinafter, examples and the like will be described in detail to help understanding of the present disclosure. However, the following examples and the like merely illustrate the contents of the present disclosure, and the scope of the present disclosure is not limited to the following examples and the like. The examples and the like of the present disclosure are provided to more completely explain the present disclosure to those having ordinary skill in the art.

### [Preparation Example] Synthesis of PRG-N-01 compound

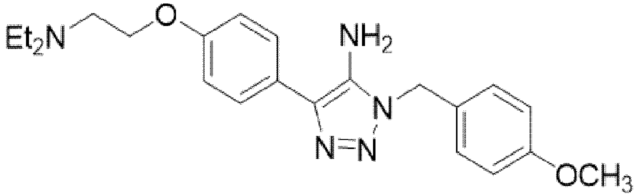

### (1) Step 1: Synthesis of Intermediate-1 (Azide)

4-MBC (4-methoxybenzyl chloride; 638.5 mmol, 100 g) was weighed and placed in a round-bottom flask, and DMF (dimethylformamide) (1,000 mL) was added thereto and dissolved. When the reactant was completely dissolved, NaN₃ (957.8 mmol, 62.3 g) was added thereto, and then the same was reacted at 25 to 30 °C for 2 hours. The reaction solution was cooled to 5 to 10 °C, and then purified water (2,500 mL) was slowly added thereto. The reaction solution was transferred to a separatory funnel, extracted with IPE (Isopropyl ether; 2,500 mL), and residual moisture was removed with anhydrous magnesium sulfate. The reaction solution was concentrated under reduced pressure and used in the next reaction without separate additional purification.
HPLC analysis result: 98.5 area%

### (2) Step 2: Synthesis of Intermediate-2 (Cyanide)

4-HPA (4-hydroxyphenylacetonitrile) (851.2 mmol, 113.3 g) was weighed and placed in a round-bottom flask, and THF (Tetrahydrofuran; 1,133 mL) was added thereto and dissolved. When the reactant was completely dissolved, DEAECl.HCl (2-Diethylaminoethyl chloride hydrochloride; 1,021.4 mmol, 175.8 g) and purified water (226 mL) were added thereto, and the reactant was cooled to 10 to 20 °C. A solution in which NaOH (2,553.5 mmol, 102.1 g) was dissolved in purified water (453 mL) was slowly added to the reaction solution, and then the temperature of the reactant was raised to 60 to 65 °C, and the same was reacted for 3 hours. The reaction solution was cooled to 20 to 30 °C, and then the reaction product was transferred to a separatory funnel to collect an organic layer, and residual moisture was removed with anhydrous magnesium sulfate. The reaction solution was concentrated under reduced pressure and used in the next reaction without separate additional purification.
HPLC analysis result: 99.2 area%

### (3) Step 3: Synthesis of crude PRG-N-01

The intermediate-1 (Azide, 625.8 mmol, 102.1 g) and intermediate-2 (Cyano, 817.1 mmol, 189.8 g) were weighed and placed in a round-bottom flask, and DMSO (706.3 mL) was added thereto and dissolved. The reactant was cooled to 10 to 15 °C, KTB (Potassium tert-Butoxide; 1,251.5 mmol, 140.4 g) was slowly added thereto, and then the temperature of the reactant was raised to 20 to 30 °C, and the same was reacted for 2 hours. The reaction solution was cooled again to 10 to 15 °C, and then purified water (1,020 mL) was slowly added thereto. The reaction solution was transferred to a separatory funnel, extracted with CH₂Cl₂ (1,020 mL), and washed with 20 % brine (280 mL). Residual moisture was removed with anhydrous magnesium sulfate. After concentration under reduced pressure, crystallization was performed using CH₂Cl₂ (150 mL) and IPE (Isopropyl ether; 2,040 mL).
HPLC analysis result: 99.1 area%
Yield: overall 80 %, off-white solid

### (4) Step 4: Synthesis of PRG-N-01

The crude PRG-N-01 (500.6 mmol, 200 g) was weighed and placed in a round-bottom flask, and CH₂Cl₂ (600 mL) was added thereto and dissolved. When the reactant was completely dissolved, activated carbon (10 wt%, 20 g) was added thereto, and then the same was stirred at a temperature of 20 to 30 °C for 1 hour. The reactant was filtered through a Celite pad and sufficiently washed with CH₂Cl₂ to recover an organic layer. After concentration under reduced pressure, crystallization was performed using CH₂Cl₂ (600 mL) and IPE (Isopropyl ether; 2,400 mL).
HPLC analysis result: 99.8 area%
Yield: 90 %, white solid
¹H-NMR (400MHz, DMSO-d₆)*δ* : 7.62(d, 2H), 7.23(d, 2H), 6.95(d, 2H), 6.91(d, 2H), 5.65(s, 2H), 5.36(s, 2H), 4.02(t, 2H), 3.72(s, 3H), 2.77(t, 2H), 2.55(q, 4H), 0.97(t, 6H)

### [Experimental Example 1] Optimization study of synthesis of Intermediate-1 (Azide)

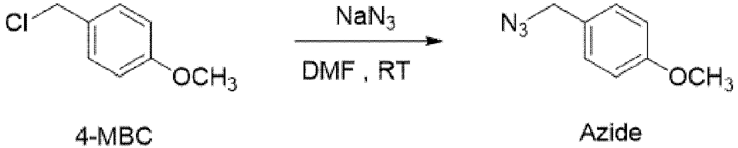

The synthesis of Intermediate-1 (Azide) was performed in the same manner as in step 1 of the above Example, and the result was analyzed by HPLC. The operating conditions and specifications of the HPLC are as follows.

Detector: ultraviolet absorption spectrometer (measurement wavelength: 226 nm)
Column: Agilent eclipse plus C8 (4.6 mm× 250 mm, 5µm)
Column temperature: maintained constant near about 40 °C
Mobile phase-A: 20 mM Sodium octane sulfonate in Water
Mobile phase-B: 0.01 % TFA in Acetonitrile

**[Table 1]**

| Time (min) | Mobile phase A (%) | Mobile phase B (%) |
|---|---|---|
| 0 | 65 | 35 |
| 5 | 65 | 35 |
| 7 | 60 | 40 |
| 20 | 60 | 40 |
| 35 | 20 | 80 |
| 43 | 20 | 80 |
| 43.1 | 65 | 65 |
| 48 | 35 | 35 |

Flow rate: 0.8 mL/min, analysis time: 48 minutes
Diluent: 100 % methanol
Sample concentration: 100 mg of a sample was placed in a 100 mL volumetric flask, and the diluent was added thereto and dissolved, followed by filling up to the mark.
Injection volume: 5 µL

### (1) Effect of inducing synthesis of Intermediate-1 (Azide) according to reaction solvent

**[Table 2]**

| Solvent | Reaction temperature | HPLC analysis (area%) | | |
|---|---|---|---|---|
| | | 4-MBC | unknown | Azide |
| DMF | 20~30 °C | trace | trace | 100 % |
| ACN | 20~30 °C | 55.09 | 14.55 | 30.36 |
| ACT | 20~30 °C | 62.66 | 19.67 | 17.67 |
| THF | 20~30 °C | 41.45 | 34.15 | 24.40 |

The reaction was performed in the same manner as in step 1 of the above Example using DMF, acetonitrile (ACN), acetone (ACT), and tetrahydrofuran (THF). When the reaction was performed using acetonitrile, acetone, and tetrahydrofuran solvents, an unknown peak occurred, whereas when the reaction was performed with the DMF solvent, no unknown peak was observed, and it could be confirmed that the reaction was completed in only 2 hours.

### (2) Effect of inducing synthesis of Intermediate-1 (Azide) according to reaction temperature

**[Table 3]**

| Reaction temperature | Solvent | HPLC analysis (area%) | | |
|---|---|---|---|---|
| | | 4-MBC | unknown | Azide |
| 5~10 °C | DMF | 6.57 | 1.91 | 89.05 |
| 15~20 °C | DMF | 0.12 | 2.11 | 95.15 |
| 25~30 °C | DMF | 0.08 | 2.05 | 95.56 |
| 35~40 °C | DMF | 0.08 | 2.00 | 95.46 |
| 45~50 °C | DMF | 0.09 | 2.07 | 95.41 |
| 55~60 °C | DMF | 0.08 | 2.05 | 95.42 |

In the same manner as in step 1 of the above Example, it was confirmed that the starting material (4-MBC) remained in the reaction temperature range of 5 to 10 °C, and that the reaction was completed by reaction for 2 hours in other temperature ranges, with no significant difference in terms of purity. Therefore, the reaction temperature was set to 25 to 30 °C, which is an appropriate reaction condition.

### (3) Effect of inducing synthesis of Intermediate-1 (Azide) according to the amount of NaN₃ used

**[Table 4]**

| Amount of NaN₃ used (eq.) | Reaction temperature | Solvent | HPLC analysis (area%) | | |
|---|---|---|---|---|---|
| | | | 4-MBC | unknown | Azide |
| 1.1 | 25~30 °C | DMF | 0.08 | 2.07 | 95.57 |
| 1.2 | 25~30 °C | DMF | 0.07 | 2.06 | 95.55 |
| 1.3 | 25~30 °C | DMF | 0.07 | 2.07 | 95.52 |
| 1.5 | 25~30 °C | DMF | 0.07 | 2.07 | 95.68 |
| 2.2 | 25~30 °C | DMF | 0.07 | 2.06 | 95.65 |

In the same manner as in step 1 of the above Example, as a result of conducting an experiment on the amount of NaN₃ used for synthesis of Intermediate-1 (Azide), it could be confirmed that, when used in the range of 1.1 to 3.0 eq, the reaction was completed within 2 hours in all cases. Theoretically, NaN₃ and 4-MBC (4-methoxybenzyl chloride) undergo a 1:1 reaction, and there is no problem even when NaN₃ is used in an excess amount of 3 eq; however, the amount of NaN₃ used was set to 1.5 equivalents, which is an intermediate range.

### [Experimental Example 2] Optimization study of synthesis of Intermediate-2 (Cyano)

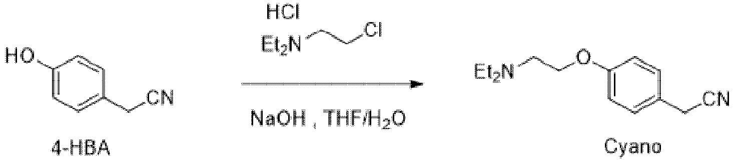

The synthesis of Intermediate-2 (Cyano) was performed in the same manner as in step 2 of the above Example, and the result was analyzed by HPLC.

### (1) Effect of inducing synthesis of Intermediate-2 (Cyano) according to reaction temperature

**[Table 5]**

| Reaction temperature | Amount of DEAECl.HCl used (eq.) | HPLC analysis (area%) | | | |
|---|---|---|---|---|---|
| | | ID | 4-HPA | Cyano | UK-2 |
| 40 ~ 45 °C | 1.2 | rxn 2h | 0.19 | 98.77 | 0.23 |
| | | rxn 3h | Trace | 98.71 | 0.28 |
| | | rxn 4h | Trace | 98.52 | 0.30 |
| 50 ~ 55 °C | 1.2 | rxn 2h | Trace | 98.24 | 0.32 |
| | | rxn 3h | Trace | 98.04 | 0.35 |
| | | rxn 4h | Trace | 98.10 | 0.30 |
| 60 ~ 65 °C | 1.2 | rxn 2h | Trace | 98.15 | 0.14 |
| | | rxn 3h | Trace | 98.03 | 0.12 |
| | | rxn 8h | trace | 97.59 | 0.20 |

In the same manner as in step 2 of the above Example, it was confirmed that the reaction was completed within 2 to 3 hours in the temperature range of 40 to 65 °C. In the reaction at 60 to 65 °C, it could be confirmed that the reaction rate at which the starting material (4-HPA) was converted into Cyano was fast and that unknown impurity-2 (hereinafter referred to as UK-2) was generated in a relatively small amount. Therefore, it could be confirmed that it is advantageous to terminate the reaction in a short time as the reaction temperature.

### (2) Effect of inducing synthesis of Intermediate-2 (Cyano) according to type of inorganic base

**[Table 6]**

| Inorganic base | Amount used (eq.) | Reaction temperature | HPLC analysis (area%) | | |
|---|---|---|---|---|---|
| | | | 4-HPA | Cyano | UK-2 |
| K₂CO₃ | 4.7 | 60 ~ 65 °C | trace | 97.00 | 1.39 |
| K₂CO₃ | 4.0 | 60 ~ 65 °C | 1.25 | 94.87 | 2.67 |
| K₂CO₃ | 3.0 | 60 ~ 65 °C | trace | 98.77 | 0.43 |
| K₂CO₃ | 2.85 | 60 ~ 65 °C | 0.95 | 96.56 | trace |
| KOH | 1.5 | 60 ~ 65 °C | 83.98 | 11.87 | 4.16 |
| KOH | 2.5 | 60 ~ 65 °C | 40.57 | 56.21 | 3.22 |
| NaOH | 1.5 | 60 ~ 65 °C | 62.21 | 32.14 | 5.65 |
| NaOH | 2.5 | 60 ~ 65 °C | 2.99 | 96.13 | 0.88 |
| 45%(w/v) NaOH solution | 3.0 | 60 ~ 65 °C | Trace | 98.26 | 0.66 |
| 22.5%(w/v) NaOH solution | 3.0 | 60 ~ 65 °C | trace | 99.27 | trace |

In the same manner as in step 2 of the above Example, when K₂CO₃ was used in an amount of 2.85 equivalents or more, there was no problem with the reaction, but there was a disadvantage in that the amount of inorganic base to be added was large. In the case of NaOH, the reaction proceeded best at 3 equivalents, and NaOH was advantageous for reaction progress due to its relatively small molecular weight. In particular, when a 22.5 %(w/v) NaOH solution was added, impurity generation at RT 12 minutes was minimized, and thus the inorganic base was set as addition of a NaOH solution.

### (3) Effect of inducing synthesis of Intermediate-2 (Cyano) according to amount of DEAECl.HCl (2-Diethylaminoethyl chloride hydrochloride) used

**[Table 7]**

| Amount of DEAECl.HCl used (eq.) | Reaction temperature | Reaction time | HPLC analysis (area%) | | |
|---|---|---|---|---|---|
| | | | 4-HPA | Cyano | UK-2 |
| 1.5 | 60 ~ 65 °C | 1.5 | Trace | 98.26 | 0.66 |
| 1.2 | 60 ~ 65 °C | 1.5 | Trace | 98.93 | 0.10 |
| 1.2 | 60 ~ 65 °C | 3.0 | trace | 99.27 | trace |
| 1.1 | 40 ~ 45 °C | 1.5 | 1.19 | 98.22 | 0.09 |
| 1.1 | 40 ~ 45 °C | 3.0 | 1.10 | 98.33 | 0.13 |
| 1.1 | 60 ~ 65 °C | 1.5 | 0.45 | 98.98 | 0.11 |
| 1.1 | 60 ~ 65 °C | 3.0 | 0.31 | 99.08 | 0.09 |

In the same manner as in step 2 of the above Example, as a result of conducting experiments while changing the amount of DEAECl.HCl used from 1.1 to 1.5 equivalents, a suitable level of result was confirmed at 1.1 equivalents only in the reaction at 65 °C. Therefore, it was confirmed that the amount of DEAECl.HCl used, which can convert all of 4-HPA into cyano and minimize generation of the UK-2 impurity, is 1.2 equivalents.

### [Experimental Example 3] Optimization study of synthesis of crude PRG-N-01

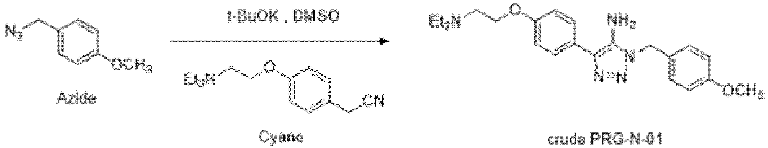

The synthesis of crude PRG-N-01 was performed in the same manner as in step 3 of the above Example, and the result was analyzed by HPLC.

### (1) Effect of inducing synthesis of crude PRG-N-01 according to reaction solvent

**[Table 8]**

| Solvent | Reaction temperature | HPLC analysis (area%) | | | |
|---|---|---|---|---|---|
| | | Cyano | PRG-N-01 | UK-3 | Azide |
| THF | 20 ~ 30 °C | 1.99 | 71.89 | 1.32 | trace |
| DMSO | 20 ~ 30 °C | 0.56 | 88.40 | 4.53 | trace |
| DMF | 20 ~ 30 °C | 0.48 | 78.39 | 4.96 | 1.59 |
| IPE-THF | 20 ~ 30 °C | 0.12 | 86.04 | 5.56 | 0.16 |

In the same manner as in step 3 of the above Example, the cyano reaction was performed using THF, DMSO, DMF, and IPE-THF solvents. When tetrahydrofuran (THF) was used as the reaction solvent, it could be confirmed that a large amount of unknown impurities was generated, resulting in low product purity, and there was a problem in that viscous brown crystals were generated after crystallization. When DMF was used as the reaction solvent, unknown impurity-3 (hereinafter referred to as UK-3) increased, resulting in overall low product purity. When DMSO was used as the reaction solvent, the purity of the product during synthesis was the best, and most of the generated impurities were removed in the work-up process, and thus the reaction solvent was set as DMSO.

### (2) Effect of inducing synthesis of crude PRG-N-01 according to type of inorganic base

**[Table 9]**

| Solvent | Reaction time | HPLC analysis (area%) | | | |
|---|---|---|---|---|---|
| | | Cyano | PRG-N-01 | UK-3 | Azide |
| KTB | 2 hours | 0.05 | 86.56 | 6.01 | 0.27 |
| NaOH | 2 hours | 9.13 | 84.42 | 2.21 | trace |
| NaOEt | 2 hours | 8.32 | 83.85 | 2.56 | 0.72 |
| KOH | 2 hours | 51.05 | 4.50 | 0.15 | 42.31 |
| DBU | 2 hours | 53.92 | trace | trace | 44.77 |

In the same manner as in step 3 of the above Example, as a result of conducting an experiment for setting an inorganic base for synthesis of crude PRG-N-01, in the case of KOH, the reactivity was significantly low, and in DBU (1,8-Diazabicyclo[5.4.0]undec-7-ene), the reaction did not proceed. Although results at a level suitable for the criteria were confirmed when the reaction was performed using NaOH and NaOEt, a problem in that stirring was difficult due to increased viscosity of the reactant was confirmed. Therefore, the inorganic base for synthesis of crude PRG-N-01 was set as KTB.

### (3) Effect of inducing synthesis of crude PRG-N-01 according to amount of KTB (Potassium tert-Butoxide; t-BuOK) used

**[Table 10]**

| Amount of KTB used (eq.) | Reaction time | HPLC analysis (area%) | | | |
|---|---|---|---|---|---|
| | | Cyano | PRG-N-01 | UK-3 | Azide |
| 1.5 | 2 hours | 1.05 | 88.41 | 4.40 | 0.14 |
| 2.0 | 2 hours | 0.05 | 86.56 | 6.01 | 0.27 |
| 2.5 | 2 hours | trace | 88.28 | 5.15 | trace |
| 3.0 | 2 hours | trace | 85.00 | 6.38 | trace |

In the same manner as in step 3 of the above Example, as a result of conducting an experiment on the amount of KTB used for synthesis of crude PRG-N-01, when KTB was used in the range of 1.5 to 3.0 eq, it was confirmed to be at a level suitable for the criteria in all cases. In addition, since UK-3 is all removed after crystallization, it was determined that there would be no problem with purity, and therefore the amount of KTB used was set to 2.0 eq.

### (4) Effect of inducing synthesis of crude PRG-N-01 according to order of adding raw materials

**[Table 11]**

| Entry | Addition order | HPLC analysis (area%) | |
|---|---|---|---|
| | | PRG-N-01 | Unknown |
| 1 | Azide, Cyano > KTB | 99.7 | - |
| 2 | Cyano > KTB > Azide | 99.2 | - |
| 3 | Azide > KTB > Cyano | 0.4 | 75.1 |

As a result of proceeding in the same manner as in step 3 of the above Example, in the case of Table 11 Entry-3, it was confirmed that an unknown side reaction occurred when the reaction was performed in the order of Azide > KTB > Cyano. In the case of Table 11 Entry-2, in which KTB was added to the cyano reactant and an Azide in DMSO solution was slowly added thereto, exotherm control was possible, but continuous exotherm was observed during the addition process of Azide in DMSO, and the final product appearance was confirmed to be yellow.

As in Table 11 Entry-1, when KTB was added to the Azide and Cyano reactants, exotherm of about 25 °C was also observed, but when initial exotherm was controlled by adding KTB in portions (1 % of the total amount to be added), it was observed that no further exotherm occurred. Therefore, the order of adding KTB to the Azide and Cyano reactants was set.

### [Experimental Example 4] Optimization study of synthesis of PRG-N-01

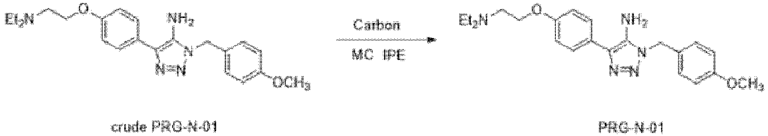

The synthesis of PRG-N-01 was performed in the same manner as in step 4 of the above Example, and the result was analyzed by HPLC.

(1) Effect of inducing purification of PRG-N-01 according to amount of CH₂Cl₂ used

**[Table 12]**

| Amount of CH₂Cl₂ used | HPLC analysis (area%) | | | | | | Yield (%) | Appearance |
|---|---|---|---|---|---|---|---|---|
| | 9.78 | 11.00 | 12.38 | 12.89 | 15.56 | 16.96 | | |
| 1.5 vol* | 0.17 | 99.30 | 0.22 | 0.10 | 0.09 | 0.13 | 96.0 | light yellow |
| 3.0 vol | 0.22 | 99.41 | 0.19 | 0.07 | 0.03 | 0.05 | 90.5 | white |
| 4.0 vol | 0.27 | 99.49 | 0.16 | 0.03 | 0.01 | 0.03 | 83.8 | white |
| 5.0 vol | 0.15 | 99.72 | 0.09 | 0.02 | 0.01 | 0.01 | 75.5 | white |
| 7.0 vol | 0.09 | 99.81 | 0.06 | 0.02 | 0.01 | 0.01 | 68.3 | white |

| | | | | | | | | |
|---|---|---|---|---|---|---|---|---|
| * vol: means that an amount corresponding to X.X times the input weight of PRG-N-01 is measured by volume and used. Example) purification of 1 kg of PRG-N-01 → 1.5 vol CH₂Cl₂ = 1.5 L | | | | | | | | |

In the same manner as in step 4 of the above Example, as a result of conducting an experiment to confirm the purification effect according to the amount of CH₂Cl₂ used, it was confirmed that, as the amount of CH₂Cl₂ used increased, when IPE was added after dissolving the reactant to induce crystal formation, color removal was better achieved. When IPE was added after dissolving in 1.5 vol, a large amount of solid was precipitated at one time, whereas when IPE was added after dissolving in CH₂Cl₂, the solid was slowly precipitated and thus was more effective in improving purity and appearance. In the case of proceeding with reflux, it was confirmed that purity was improved, but there was a disadvantage in that yield was greatly reduced. Therefore, when purifying crude PRG-N-01, the amount of CH₂Cl₂ used was set to 3 vol, which is an intermediate value of 1.5 to 4 vol.

### (2) Effect of inducing purification of PRG-N-01 according to type of activated carbon

**[Table 13]**

| Type of activated carbon | HPLC analysis (area%) | | | | | | Appearance |
|---|---|---|---|---|---|---|---|
| | 9.78 | 11.00 | 12.38 | 12.89 | 15.56 | 16.96 | |
| Crude (solution) | 0.10 | 98.62 | 0.39 | 0.19 | 0.34 | 0.29 | - |
| Shinki carbon (reaction solution) | 0.09 | 99.03 | 0.12 | 0.04 | 0.32 | 0.30 | - |
| Shinki carbon solid | 0.10 | 99.61 | 0.11 | trace | 0.05 | 0.08 | white |
| Neutral carbon (reaction solution) | 0.10 | 99.04 | 0.12 | 0.05 | 0.34 | 0.29 | - |
| Neutral carbon solid | 0.09 | 99.54 | 0.13 | trace | 0.05 | 0.10 | white |
| SA-20 (reaction solution) | 0.10 | 98.91 | 0.16 | 0.11 | 0.34 | 0.29 | - |
| SA-20 solid | 0.09 | 99.53 | 0.16 | trace | 0.04 | 0.10 | white |

In the same manner as in step 4 of the above Example, as a result of conducting an experiment to confirm the purification effect according to the type of activated carbon, it was confirmed that impurities at RT 12.38 minutes were removed by carbon (impurity carbon) treatment in all of Shinki carbon (Shinki Chemical Industry; Korea), neutral carbon (Norit; Japan), and SA-20 (MEADWESTVACO; USA). Among these, it was confirmed that the purity after Shinki carbon treatment was the best, and thus purification using Shinki carbon was set.

The foregoing description of the present disclosure is for illustration, and those having ordinary skill in the art to which the present disclosure pertains will understand that the present disclosure can be easily modified into other specific forms without changing the technical spirit or essential features of the present disclosure. Therefore, it should be understood that the embodiments described above are illustrative in all respects and not restrictive.

The scope of the present disclosure is indicated by the claims described below, and all changes or modified forms derived from the meaning and scope of the claims and the equivalent concept thereof should be construed as being included in the scope of the present disclosure.

## Claims

1. A method for preparing 4-(4-(2-(diethylamino)ethoxy)phenyl)-1-(4-methoxybenzyl)-1H-1,2,3-triazole-5-amine, comprising:
a) dissolving 4-MBC (4-methoxybenzyl chloride) in DMF (dimethylformamide), and then adding NaN₃ (Sodium azide) thereto and reacting the same for 1 to 3 hours to synthesize an intermediate;
b-1) dissolving 4-HPA (4-hydroxyphenylacetonitrile) in THF (tetrahydrofuran), and then adding DEAECl.HCl (2-Diethylaminoethyl chloride hydrochloride) and water thereto and cooling the same to 5 to 20 °C;
b-2) adding an aqueous NaOH solution to the reaction product of step b-1) and reacting the same for 2 to 4 hours to synthesize an intermediate;
c) dissolving the intermediate of step a) and the intermediate of step b-2) in DMSO, and then adding KTB (Potassium tert-Butoxide; t-BuOK) thereto and reacting the same at 20 to 40 °C for 1 to 3 hours; and
d) dissolving the reaction product of step c) in CH₂Cl₂ (dichloromethane), adding activated carbon thereto, stirring the same at 15 to 40 °C for 30 to 100 minutes, and filtering and washing the same.

2. The method of claim 1, wherein the reaction of step a) is performed at 15 to 60 °C.

3. The method of claim 1, wherein the amount of NaN₃ used in step a) is 1.0 to 3.0 eq.

4. The method of claim 1, wherein the amount of DEAECl.HCl used in step b-1) is 1.0 to 1.5 eq.

5. The method of claim 1, wherein the reaction of step b-2) is performed at 40 to 70 °C.

6. The method of claim 1, wherein the amount of NaOH used in step b-2) is 2.5 to 4 eq.

7. The method of claim 1, wherein the aqueous NaOH solution of step b-2) is a 20 to 50 %(w/v) aqueous NaOH solution.

8. The method of claim 1, wherein the amount of KTB used in step c) is 1.0 to 3.0 eq.

9. The method of claim 1, wherein, in step d), a volume ratio of the reaction product of step c) to CH₂Cl₂ (dichloromethane) is 1 : 1.5 to 4.0.
